# DEMANDE DE BREVET EUROPEEN

(11) **EP 1 157 684 A2**
(43) Date de publication de la demande: **28.11.2001**
(21) Numéro de dépôt: 01401171.2
(22) Date de dépôt: 07.05.2001
(51) Int. Cl.: A61K 7/13, A61K 7/135, A61K 7/09, A61K 7/06

(54) **Composition oxydante et utilisations pour la teinture, pour la déformation permanente ou pour la décoloration des fibres kératiniques**

(30) Priorité: 15.05.2000 FR 0006153
(71) Demandeur: L'OREAL, 75008 Paris (FR)
(72) Inventeur: Kravtchenko, Sylvain, 92600 Asnieres (FR); Plos, Gregory, 75011 Paris (FR)
(74) Mandataire: Miszputen, Laurent

(57) **Abrégé**

La présente demande concerne une composition oxydante cosmétique destinée au traitement des fibres kératiniques, en particulier des fibres kératiniques humaines et plus particulièrement les cheveux humains, comprenant dans un milieu approprié pour les fibres kératiniques :
(a) au moins un système oxydant enzymatique comprenant au moins une enzyme de type oxydo-réductase à 2 électrons ou à 4 électrons ou peroxydase,
(b) au moins un copolymère réticulé anhydride maléique / alkyl(C₁-C₅) vinyl éther;
ainsi qu'un procédé pour améliorer la conservation de l'activité enzymatique d'une composition oxydante destinée à la teinture, la déformation permanente ou la décoloration des fibres kératiniques, en particulier des cheveux humains.

## Description

La présente invention concerne une composition oxydante, destinée au traitement des fibres kératiniques comprenant au moins un système oxydant enzymatique comprenant au moins une enzyme de type oxydo-réductase à 2 électrons ou à 4 électrons ou peroxydase et au moins un copolymère réticulé anhydride maléique / alkyl(C₁-C₅) vinyl éther, ainsi que ses utilisations pour la teinture, pour la déformation permanente ou pour la décoloration des fibres kératiniques en particulier des cheveux humains.

Il est connu de teindre les fibres kératiniques et en particulier les cheveux humains avec des compositions tinctoriales contenant des précurseurs de colorant d'oxydation, appelés généralement bases d'oxydation, en particulier des paraphénylènediamines, des ortho- ou para-aminophénols, ou des bases hétérocycliques. Les précurseurs de colorants d'oxydation, ou bases d'oxydation, sont des composés incolores ou faiblement colorés qui, associés à des produits oxydants, peuvent donner naissance par un processus de condensation oxydative à des composés colorés et colorants.
On sait également que l'on peut faire varier les nuances obtenues avec ces bases d'oxydation en les associant à des coupleurs ou modificateurs de coloration, ces derniers étant choisis notamment parmi les métadiamines aromatiques, les métaaminophénols, les métadiphénols et certains composés hétérocycliques.
La variété des molécules mises en jeu au niveau des bases d'oxydation et des coupleurs, permet l'obtention d'une riche palette de couleurs.

La coloration dite "permanente" obtenue grâce à ces colorants d'oxydation, doit par ailleurs satisfaire un certain nombre d'exigences. Ainsi, elle doit être sans inconvénient sur le plan toxicologique, elle doit permettre d'obtenir des nuances dans l'intensité souhaitée et présenter une bonne tenue face aux agents extérieurs (lumière, intempéries, lavage, ondulation permanente, transpiration, frottements).

Les colorants doivent également permettre de couvrir les cheveux blancs, et être enfin les moins sélectifs possible, c'est à dire permettre d'obtenir des écarts de coloration les plus faibles possible tout au long d'une même fibre kératinique, qui peut être en effet différemment sensibilisée (i.e. abîmée) entre sa pointe et sa racine.

La coloration d'oxydation des fibres kératiniques est généralement réalisée en milieu alcalin, en présence de peroxyde d'hydrogène comme oxydant. Toutefois, l'utilisation des milieux alcalins en présence de peroxyde d'hydrogène présentent, pour inconvénient, d'entraîner une dégradation non négligeable des fibres, ainsi qu'une décoloration importante des fibres kératiniques qui n'est pas toujours souhaitable.

La coloration d'oxydation des fibres kératiniques peut également être réalisée à l'aide de systèmes oxydants différents du peroxyde d'hydrogène tels que des systèmes enzymatiques.
Ainsi il a déjà été proposé de teindre les fibres kératiniques, notamment dans la demande de brevet EP-A-0 310 675, avec des compositions comprenant un précurseur de colorant d'oxydation en association avec des enzymes telles que la pyranose-oxydase, la glucose-oxydase ou bien l'uricase, en présence d'un donneur pour lesdites enzymes. Ces formulations de teinture, bien qu'étant mises en oeuvre dans des conditions n'entraînant pas une dégradation des fibres kératiniques comparable à celle engendrée par les teintures réalisées en présence de peroxyde d'hydrogène, conduisent à des colorations encore insuffisantes, à la fois sur le plan de l'homogénéité de la couleur répartie le long de la fibre (« unisson »), sur le plan de la chromaticité (luminosité), de la puissance tinctoriale et de la résistance vis à vis des diverses agressions que peuvent subir les cheveux.

On sait que la technique la plus usuelle pour obtenir une déformation permanente des cheveux consiste, dans un premier temps, à réaliser l'ouverture des liaisons disulfures -S-S- de la kératine (cystine) à l'aide d'une composition contenant un agent réducteur adapté (étape de réduction), puis, après avoir rincé la chevelure ainsi traitée, à reconstituer dans un second temps lesdites liaisons disulfures, en appliquant sur les cheveux préalablement mis sous tension (bigoudis et autres), une composition oxydante (étape d'oxydation, dite aussi de fixation) de façon à donner finalement aux cheveux la forme recherchée. Cette technique permet ainsi de réaliser indifféremment, soit l'ondulation des cheveux, soit leur défrisage ou leur décrêpage. La nouvelle forme imposée aux cheveux par un traitement chimique tel que ci-dessus est éminemment durable dans le temps et résiste notamment à l'action des lavages à l'eau ou aux shampooings, et ceci par opposition aux simples techniques classiques de déformation temporaire, telles que de mise en pli.
Les compositions réductrices utilisables pour la mise en oeuvre de la première étape d'une opération de permanente contiennent généralement, à titre d'agents réducteurs, des sulfites, des bisulfites, des alkyl-phosphines ou de préférence des thiols. Parmi ces derniers, ceux couramment utilisés sont la cystéïne et ses dérivés, la cystéamine et ses dérivés, l'acide thiolactique ou l'acide thioglycolique, leurs sels ainsi que leurs esters, notamment le thioglycolate de glycérol.
Concernant les compositions oxydantes nécessaires à la mise en oeuvre de l'étape de fixation, on fait le plus souvent appel, dans la pratique, à des compositions à base d'eau oxygénée, de bromate de sodium ou de persels comme le perborate de sodium, qui ont le désavantage d'être susceptibles d'abîmer les cheveux.
Le problème de la technique des permanentes connues à ce jour est que leur application sur les cheveux induit à la longue une altération de la qualité des cheveux. Les causes essentielles de cette altération de la qualité des cheveux sont une diminution de leurs propriétés cosmétiques, telles que leur brillance, le toucher et une dégradation de leurs propriétés mécaniques, plus particulièrement une dégradation de leur résistance mécanique due à un gonflement des fibres kératiniques lors du rinçage entre l'étape de réduction et l'étape d'oxydation et qui peut également se traduire par une augmentation de leur porosité. Les cheveux sont affaiblis et peuvent devenir cassants lors de traitements ultérieurs comme des brushings.

On retrouve le même problème d'altération de la fibre kératinique lors des procédés de décoloration des cheveux.

On sait que la déformation permanente ou la décoloration des fibres kératiniques peut également être réalisée dans des conditions plus douces à l'aide de systèmes oxydants différents du peroxyde d'hydrogène tels que des systèmes enzymatiques.
Ainsi il a déjà été proposé des procédés de déformation permanente ou de décoloration des fibres kératiniques, notamment dans la demande de brevet EP-A-0 310 675, avec des compositions comprenant une enzyme telle que la pyranose-oxydase, la glucose-oxydase ou bien l'uricase, en présence d'un donneur pour ladite enzyme. Ces formulations oxydantes, bien qu'étant mises en oeuvre dans des conditions n'entraînant pas une dégradation des fibres kératiniques comparable à celle engendrée par les procédés classiques de permanente ou de décoloration, conduisent à des résultats encore insuffisants quant à la tenue de la frisure dans le temps, quant à la compatibilité des cheveux permanentés ou décolorés avec les traitements ultérieurs, quant à la réduction de la dégradation des propriétés mécaniques des cheveux permanentés notamment la réduction de la porosité des cheveux, quant aux propriétés cosmétiques telles que le toucher ou bien encore sur le plan de l'uniformité de la décoloration le long des fibres kératiniques.

En outre, on a constaté que les épaississants généralement utilisés dans ce type de composition oxydante enzymatique de l'art antérieur pour mieux la localiser au point d'application ne permettent pas une conservation de l'activité enzymatique suffisamment prolongée dans le temps.

La présente invention a pour but de résoudre les problèmes évoqués ci-dessus.

La Demanderesse a découvert de façon tout à fait inattendue et surprenante qu' en utilisant un copolymère réticulé anhydride maléique / alkyl(C₁-C₅) vinyl éther, dans une composition oxydante enzymatique comprenant au moins une oxydo-réductase à 2 ou à 4 électrons ou une peroxydase, il s'est avéré possible d'obtenir une meilleure conservation de l'activité enzymatique au cours du temps.

Elle a également découvert de nouvelles compositions contenant au moins un système oxydant enzymatique comprenant au moins une enzyme de type oxydo-réductase à 2 électrons ou à 4 électrons ou peroxydase et au moins un copolymère réticulé anhydride maléique / alkyl(C₁-C₅) vinyl éther, qui peuvent constituer en présence de colorants d'oxydation (bases d'oxydation et/ou coupleurs), des formulations de teinture prêtes à l'emploi, conduisant à des colorations plus homogènes, au moins aussi puissantes et aussi chromatiques que celles de l'art antérieur et sans engendrer de dégradation significative, peu sélectives et résistant bien aux diverses agressions que peuvent subir les cheveux (lumière, intempéries, lavage, ondulation permanente, transpiration, frottements).

Elle a découvert aussi que l'utilisation, dans un procédé de déformation permanente des fibres kératiniques, d'une composition oxydante contenant au moins un système oxydant enzymatique comprenant au moins une enzyme de type oxydo-réductase à 2 électrons ou à 4 électrons ou peroxydase et au moins un copolymère réticulé anhydride maléique / alkyl(C₁-C₅) vinyl éther, permettait de résoudre les problèmes techniques évoqués ci-dessus. En particulier, ce type de composition oxydante permet d'améliorer la tenue à la frisure obtenue dans le temps, de réduire sensiblement la porosité des cheveux permanentés, d'améliorer la compatibilité des cheveux permanentés aux traitements ultérieurs.

La Demanderesse a découvert en outre de façon surprenante que l'utilisation, dans un procédé de décoloration des fibres kératiniques, d'une composition oxydante contenant au moins un système oxydant enzymatique comprenant au moins une enzyme de type oxydo-réductase à 2 électrons ou à 4 électrons ou peroxydase et au moins un copolymère réticulé anhydride maléique / alkyl(C₁-C₅) vinyl éther, permettait de résoudre les problèmes techniques évoqués ci-dessus notamment d'améliorer la compatibilité des cheveux décolorés aux traitements ultérieurs. Ce type de composition oxydante permet d'obtenir un effet décolorant plus uniforme sur les cheveux et d'améliorer les propriétés cosmétiques, comme le toucher.

Ces découvertes sont à la base de la présente invention.

La présente invention a donc pour premier objet une composition oxydante cosmétique destinée au traitement des fibres kératiniques, en particulier des fibres kératiniques humaines et plus particulièrement des cheveux humains, comprenant dans un milieu approprié pour les fibres kératiniques :
(a) au moins un système oxydant enzymatique comprenant au moins une enzyme de type oxydo-réductase à 2 électrons ou à 4 électrons ou peroxydase,
(b) au moins un copolymère réticulé anhydride maléique / alkyl(C₁-C₅) vinyl éther.

Elle a également pour objet un procédé pour améliorer la conservation de l'activité enzymatique d'une composition oxydante destinée à la teinture, la déformation permanente ou la décoloration des fibres kératiniques, en particulier des cheveux humains, et comprenant à titre de système oxydant au moins une oxydo-réductase à 2 ou à 4 électrons ou une peroxydase, caractérisé par le fait qu'on ajoute à ladite composition oxydante une quantité efficace d'un copolymère réticulé anhydride maléique / alkyl(C₁-C₅) vinyl éther.
Un procédé préféré selon l'invention comprend une oxydo-réductase de type uricase et de l'acide urique comme donneur et un copolymère réticulé anhydride maléique / méthyl vinyl éther réticulé par du 1,9-décadiène.

### Système oxydant enzymatique

### Oxydo-réductases à 2 électrons ou à 4 électrons ou peroxydases.

La ou les oxydo-réductases à 2 électrons utilisées dans la composition oxydante conforme à l'invention, sont utilisées en présence d'un donneur pour la ou lesdites enzymes, et peuvent notamment être choisies parmi les pyranose oxydases, les glucose oxydases, les glycérol oxydases, les lactate oxydases, les pyruvate oxydases, les uricases, les choline oxydases, les sarcosine oxydases, les bilirubine oxydases et les aminoacides oxydases.
Selon l'invention, l'oxydo-réductase à 2 électrons est de préférence choisie parmi les uricases d'origine animale, microbiologique ou biotechnologique.
A titre d'exemple, on peut notamment citer l'uricase extraite de foie de sanglier, l'uricase d'Arthrobacter globiformis, ainsi que l'uricase d'Aspergillus flavus.

La ou les oxydo-réductases à 2 électrons peuvent être utilisées sous forme cristalline pure ou sous une forme diluée dans un diluant inerte pour ladite oxydo-réductase à 2 électrons.

La ou les oxydo-réductases à 2 électrons conformes à l'invention représentent de préférence de 0,01 à 20% en poids environ du poids total de la composition de teinture, ou de déformation permanente ou de décoloration, et encore plus préférentiellement de 0,1 à 5% en poids environ de ce poids.

On peut aussi définir la quantité d'enzyme en fonction de son activité.
L'activité enzymatique des oxydoréductases à 2 électrons conformes à l'invention peut être définie à partir de l'oxydation du donneur en condition aérobie.
Une unité U correspond à la quantité d'enzyme conduisant à la génération d'une µmole de H₂O₂ par minute à un pH de 8,5 et à une température de 25°C. De façon préférentielle, la quantité d'oxydoréductase à 2 électrons conforme à l'invention est comprise entre 10 et 10⁸ unités U environ pour 100g de composition de teinture, ou de déformation permanente ou de décoloration.

Selon l'invention, on entend par donneur, les différents substrats également nécessaires au fonctionnement de ladite ou desdites oxydo-réductases à 2 électrons.
La nature du donneur (ou substrat) pour ladite enzyme varie en fonction de la nature de l'oxydo-réductase à 2 électrons qui est utilisée. Par exemple, à titre de donneur pour les pyranose oxydases, on peut citer le D-glucose, le L-sorbose et le D-xylose ; à titre de donneur pour les glucose oxydases, on peut citer le D-glucose; à titre de donneur pour les glycérol oxydases, on peut citer le glycérol et la dihydroxyacétone ; à titre de donneur pour les lactate oxydases, on peut citer l'acide lactique et ses sels ; à titre de donneur pour les pyruvate oxydases, on peut citer l'acide pyruvique et ses sels ; à titre de donneur pour les uricases, on peut citer l'acide urique et ses sels ; à titre de donneur pour les choline oxydases, on peut citer la choline et ses sels d'addition avec un acide comme le chlorhydrate de choline, et la bétaïne aldéhyde ; à titre de donneur pour les sarcosine oxydases, on peut citer la sarcosine, la N-méthyl-L-leucine, la N-méthyl-DL-alanine, et la N-méthyl-DL-valine ; et enfin, à titre de donneur pour les bilirubine oxydases, on peut citer la bilirubine.

Le ou les donneurs (ou substrats) utilisés conformément à l'invention représentent de préférence de 0,01 à 20% en poids environ du poids total de la composition de teinture, ou de déformation permanente ou de décoloration conforme à l'invention et encore plus préférentiellement de 0,1 à 5% en environ de ce poids.

La ou les oxydo-réductases à 4 électrons utilisées dans la composition conforme à l'invention peuvent notamment être choisies parmi les laccases, les tyrosinases, les catéchol oxydases et les polyphénols oxydases.
Selon une forme de réalisation particulière et préférée de l'invention la ou les oxydo-réductases à 4 électrons sont choisies parmi les laccases.
Ces laccases peuvent notamment être choisies parmi les laccases d'origine végétale, d'origine animale, d'origine fongique (levures, moisissures, champignons) ou d'origine bactérienne, les organismes d'origine pouvant être mono- ou pluri- cellulaires. Les laccases peuvent également être obtenues par biotechnologie.
Parmi les laccases d'origine végétale utilisables selon l'invention, on peut citer les laccases produites par des végétaux effectuant la synthèse chlorophyllienne telles que celles indiquées dans la demande de brevet FR-A-2 694 018.
On peut notamment citer les laccases présentes dans les extraits d'Anacardiacées tels que par exemple les extraits de Mangifera indica, de Schinus molle ou de Pleiogynium timoriense ; dans les extraits de Podocarpacées ; de Rosmarinus off. ; de Solanum tuberosum ; d'Iris sp. ; de Coffea sp. ; de Daucus carrota ; de Vinca minor ; de Persea americana ; de Catharenthus roseus ; de Musa sp. ; de Malus pumila ; de Gingko biloba ; de Monotropa hypopithys (sucepin), d'Aesculus sp. ; d'Acer pseudoplatanus ; de Prunus persica et de Pistacia palaestina.
Parmi les laccases d'origine fongique, éventuellement obtenues par biotechnologie, utilisables selon l'invention, on peut citer la ou les laccases issues de Polyporus versicolor, de Rhizoctonia praticola et de Rhus vernicifera telles que décrites par exemples dans les demandes de brevet FR-A-2 112 549 et EP-A-504005 ; les laccases décrites dans les demandes de brevet WO95/07988, WO95/33836, WO95/33837, WO96/00290, WO97/19998 et WO97/19999, dont le contenu fait partie intégrante de la présente description comme par exemple la ou les laccases issues de Scytalidium, de Polyporus pinsitus, de Myceliophtora thermophila, de Rhizoctonia solani, de Pyricularia orizae, et leurs variantes. On peut également citer la ou les laccases issues de Trametes versicolor, de Fomes fomentarius, de Chaetomium thermophile, de Neurospora crassa, de Colorius versicol, de Botrytis cinerea, de Rigidoporus lignosus, de Phellinus noxius, de Pleurotus ostreatus, d'Aspergillus nidulans, de Podospora anserina, d'Agaricus bisporus, de Ganoderma lucidum, de Glomerella cingulata, de Lactarius piperatus, de Russula delica, d'Heterobasidion annosum, de Thelephora terrestris, de Cladosporium cladosporioides, de Cerrena unicolor, de Coriolus hirsutus, de Ceriporiopsis subvermispora, de Coprinus cinereus, de Panaeolus papilionaceus, de Panaeolus sphinctrinus, de Schizophyllum commune, de Dichomitus squalens, et de leurs variantes.

On choisira plus préférentiellement les laccases d'origine fongique, éventuellement obtenues par biotechnologie.
L'activité enzymatique des laccases utilisées conformément à l'invention et ayant la syringaldazine parmi leurs substrats peut être définie à partir de l'oxydation de la syringaldazine en condition aérobie. L'unité Lacu correspond à la quantité d'enzyme catalysant la conversion de 1 mmole de syringaldazine par minute à un pH de 5,5 et à une température de 30°C. L'unité U correspond à la quantité d'enzyme produisant un delta d'absorbance de 0,001 par minute, à une longueur d'onde de 530 nm, en utilisant la syringaldazine comme substrat, à 30°C et à un pH de 6,5. L'activité enzymatique des laccases utilisées selon l'invention peut aussi être définie à partir de l'oxydation de la paraphénylènediamine. L'unité ulac correspond à la quantité d'enzyme produisant un delta d'absorbance de 0,001 par minute, à une longueur d'onde de 496,5 nm, en utilisant la paraphénylènediamine comme substrat (64 mM), à 30°C et à un pH de 5.

De manière générale, la ou les oxydo-réductases à 4 électrons conformes à l'invention représentent de préférence de 0,01 à 20% en poids environ du poids total de la composition de teinture, ou de déformation permanente ou de décoloration, et encore plus préférentiellement de 0,1 à 5% en poids environ de ce poids.

De manière particulière, et lorsqu'une ou plusieurs laccases sont utilisées, la quantité de laccase(s) présente dans la composition de teinture, ou de déformation permanente ou de décoloration conforme à l'invention variera en fonction de la nature de la ou des laccases utilisées. De façon préférentielle, la quantité de laccase(s) est comprise entre 0,5 et 2000 Lacu environ (soit entre 10000 et 40.10⁶ unités U environ ou soit entre 20 et 20.10⁶ unités ulac) pour 100 g de composition de teinture, ou de déformation permanente ou de décoloration.

La ou les peroxydases utilisées dans la composition de teinture, ou de déformation permanente ou de décoloration, ou de déformation permanente ou de décoloration conforme à l'invention, peuvent notamment être choisies parmi les enzymes appartenant à la sous-classe 1.11.1 décrite dans l'ouvrage Enzyme Nomenclature, Academic Press Inc., 1984. Certaines d'entre elles nécessitent la présence d'un donneur pour fonctionner. C'est le cas, en particulier des NADH peroxydases (1.11.1) [donneur = NADH], des acides gras peroxydases (1.11.1.3) [donneur = acide gras, par exemple palmitate] , des NADPH peroxydases (1.11.1.2), [donneur = NADPH], des cytochrome-c peroxydases (1.11.1.5) [donneur = ferrocytochrome c], des iodures peroxydases (1.11.1.8) [donneur = iodure], des chlorures peroxydases (1.11.10) [donneur = chlorure], des L. ascorbates peroxydases (1.11.1.11) [donneur = L.ascorbate], des glutathion peroxydases (1.11.1.9) [donneur = glutathion].
D'autres peroxydases fonctionnent sans donneur ; il en est ainsi des catalases (1.11.1.6) et des peroxydases simplex (1.11.1.7).

Selon l'invention, on utilise de préférence les peroxydases simplex (1.11.1.7).

Toutes les peroxydases fonctionnent en présence de peroxyde d'hydrogène qui est apporté tel quel ou généré in situ par voie enzymatique [oxydase(s) à 2 électrons et donneur(s) en présence d'air].

Les peroxydases utilisées peuvent être d'origine végétale, animale, fongique, bactérienne. Elles peuvent être également obtenues par biotechnologie.

Ainsi, les peroxydases peuvent par exemple être issues de la pomme, de l'abricot, de l'orge, du radis noir, de la betterave, du chou, de la carotte, du maïs, du coton, de l'ail, du raisin, de la menthe, de la rhubarbe, du soja, de l'épinard, du coprin, du lait de bovin, des microorganismes du type Acetobacter peroxidans, Staphylococcus faecalis, Arthromyces ramosus.

L'unité d'activité de la peroxydase simplex (1.11.1.7) peut se définir comme étant la quantité d'enzyme simplex formant 1mg de purpurogalline à partir du pyrogallol en 20s à pH6 et à 20°C. A titre d'exemple, la peroxydase de radis noir P6782 de SIGMA présente une activité d'environ 250 unités par mg.
La concentration d'utilisation de ce type d'enzyme varie donc entre 25 et 5.10⁶ unités pour 100g de composition.

La ou les peroxydases conformes à l'invention représentent de préférence de 0,0001 à 20% en poids environ du poids total de la composition de teinture, ou de déformation permanente ou de décoloration, et encore plus préférentiellement de 0,001 à 10% en poids environ de ce poids.

### Copolymères réticulés anhydride maléique / alkyl(C₁-C₅) vinyl éther

Les gels obtenus à partir de copolymères réticulés anhydride maléique / alkyl(C₁-C₅) vinyl éther, hydrolysés et neutralisés, sont connus dans les compositions cosmétiques pour le soin ou le traitement des cheveux ou de la peau. Ils sont décrits dans les brevets US-5034220, 5032391, 5024779 et 5254636 de la société GAF Chemicals Corporation. Le procédé de polymérisation conduisant à la synthèse de ces polymères est également décrit dans le brevet US-5034488 de ladite société.

Les copolymères réticulés anhydride maléique / alkyl(C₁-C₅) vinyl éther sont préparés par polymérisation d'anhydride maléique, d'un alkylvinyléther et d'un agent réticulant, en présence d'un initiateur de radicaux libres, dans un solvant approprié.
De préférence, à titre de solvant, on utilise un système cosolvant comprenant environ 45 à 65% de cyclohexane et environ 35-55% en poids d'acétate d'éthyle.

La polymérisation est conduite à une température comprise entre 0° et 150°C, de préférence entre 50° et 100°C et plus particulièrement entre 60° et 80°C. La quantité d'agent réticulant varie généralement d'environ 1 à environ 5 moles par rapport au monoalkylvinyléther.
Ces agents réticulants sont par exemple choisis parmi les divinyléthers de diols aliphatiques et les divinyléthers de polyéthylèneglycols et aussi parmi les 1,7-octadiène, 1,9-décadiène, divinylbenzène, N,N'-bis-méthylène acrylamide, diacrylate de polyéthylène glycol ou de propylène glycol, triacrylate de triméthylolpropane, et les alcools polyhydriques estérifiés par l'acide acrylique. On utilise plus particulièrement le 1,9-décadiène.
Les initiateurs de radicaux libres, utilisés de préférence dans des proportions comprises entre 0,001 et 1% en poids par rapport aux monomères, sont notamment choisis parmi l'azobis-isobutyronitrile, l'azobis-(2,4-diméthylvaléronitrile), ou les peroxydes de benzoyle, de lauroyle, de caprylyle, d'acétyle, d'acétylbenzoyle ou de di-tertio-butyle ou les mélanges de ces composés.
Les copolymères ainsi obtenus après filtration et séchage sont sous forme de poudre.
Ils sont hydrolysés et neutralisés dans une solution aqueuse basique telle qu'une solution aqueuse de soude ou de potasse à 10%, ou d'ammoniaque à 30%, ou d'une alcanolamine comme la monoéthanolamine, la diéthanolamine, la triéthanolamine, l'aminométhylpropanol, l'aminométhylpropanediol.

Selon la présente invention, de façon préférentielle, la viscosité d'une solution aqueuse contenant de 0,5 à 1% en poids dudit copolymère hydrolysé et neutralisé, à un pH compris entre 5 et 10, et mesurée à 25°C au viscosimètre BROOKFIELD RTV, broche 7 à 20 tours/minute, est comprise entre 45000 et 70000 mPa.s.
Les copolymères réticulés anhydride maléique / alkyl(C₁-C₅) vinyl éther préférés selon la présente invention présentent en outre une taille de particules inférieure à 850 microns, de préférence inférieure à 75 microns et plus particulièrement comprise entre 1 et 15 microns.
Un copolymère réticulé anhydride maléique / alkyl(C₁-C₅) vinyl éther particulièrement préféré selon l'invention est par exemple le copolymère anhydride maléique / méthyl vinyl éther, réticulé avec le 1,9-décadiène, dont une solution aqueuse à 0,5% en poids à pH 7 présente une viscosité à 25°C mesurée au viscosimètre BROOKFIELD RTV, broche 7 à 20 tours/minute comprise entre 45000 et 70000 mPa.s. avec une taille des particules inférieure à 75 microns, et qui est vendu sous la dénomination STABILEZE QM par la société I.S.F.

Dans la composition selon la présente invention, la concentration en copolymère réticulé anhydride maléique / alkyl(C₁-C₅) vinyl éther peut varier de 0,001 % à 10 % en poids environ par rapport au poids total de la composition, et de préférence entre 0,01 et 5 % environ.

La présente invention a également pour objet une composition prête à l'emploi, pour la teinture d'oxydation des fibres kératiniques et en particulier des fibres kératiniques humaines telles que les cheveux, du type comprenant, dans un milieu approprié pour la teinture, au moins un colorant d'oxydation, et qui est caractérisée par le fait qu'elle contient :
(a) au moins un système oxydant enzymatique comprenant au moins une enzyme de type oxydo-réductase à 2 électrons ou à 4 électrons ou peroxydase,
(b) au moins un copolymère réticulé anhydride maléique / alkyl(C₁-C₅) vinyl éther.

Les colorants d'oxydation utilisables selon l'invention sont choisis parmi les bases d'oxydation et/ou les coupleurs.
De préférence les compositions de teinture prêtes à l'emploi selon l'invention contiennent au moins une base d'oxydation.
La nature de ces bases d'oxydation n'est pas critique. Elles peuvent notamment être choisies parmi les ortho- et para-phénylènediamines, les bases doubles, les ortho- et para-aminophénols, les bases hétérocycliques suivantes ainsi que les sels d'addition de tous ces composés avec un acide.
On peut notamment citer :
- **(I)** les paraphénylènediamines de formule (I) suivante et leurs sels d'addition avec un acide : 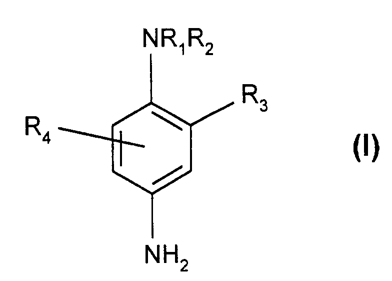 dans laquelle :
   **R**_{**1**} représente un atome d'hydrogène, un radical alkyle en C₁-C₄, monohydroxyalkyle en C₁-C₄, polyhydroxyalkyle en C₂-C₄ alcoxy(C₁-C₄)alkyle(C₁-C₄), alkyle en C₁-C₄ substitué par un groupement azoté, phényle ou 4'-aminophényle ;
   **R**_{**2**} représente un atome d'hydrogène, un radical alkyle en C₁-C₄, monohydroxyalkyle en C₁-C₄ ou polyhydroxyalkyle en C₂-C₄, alcoxy(C₁-C₄)alkyle(C₁-C₄) ou alkyle en C₁-C₄ substitué par un groupement azoté ;
   **R**_{**3**} représente un atome d'hydrogène, un atome d'halogène tel qu'un atome de chlore, un radical alkyle en C₁-C₄, sulfo, carboxy, monohydroxyalkyle en C₁-C₄ ou hydroxyalcoxy en C₁-C₄, acétylaminoalcoxy en C₁-C₄, mésylaminoalcoxy en C₁-C₄ ou carbamoylaminoalcoxy en C₁-C₄,
   **R**_{**4**} représente un atome d'hydrogène, d'halogène ou un radical alkyle en C₁-C₄.

Parmi les groupements azotés de la formule (I) ci-dessus, on peut citer notamment les radicaux amino, monoalkyl(C₁-C₄)amino, dialkyl(C₁-C₄)amino, trialkyl(C₁-C₄)amino, monohydroxyalkyl(C₁-C₄)amino, imidazolinium et ammonium.

Parmi les paraphénylènediamines de formule (I) ci-dessus, on peut plus particulièrement citer la paraphénylènediamine, la paratoluylènediamine, la 2-chloro-paraphénylènediamine, la 2,3-diméthyl-paraphénylènediamine, la 2,6-diméthyl-paraphénylènediamine, la 2,6-diéthyl-paraphénylènediamine, la 2,5-diméthyl-paraphénylènediamine, la N,N-diméthyl-paraphénylènediamine, la N,N-diéthyl-paraphénylènediamine, la N,N-dipropyl-paraphénylènediamine, la 4-amino-N,N-diéthyl-3-méthyl-aniline, la N,N-bis-(β-hydroxyéthyl)-paraphénylènediamine, la 4-N,N-bis-(β-hydroxyéthyl)amino-2-méthyl-aniline, la 4-N,N-bis-(β-hydroxyéthyl)-amino 2-chloro-aniline, la 2-β-hydroxyéthylparaphénylènediamine, la 2-fluoro-paraphénylènediamine, la 2-isopropylparaphénylènediamine, la N-(β-hydroxypropyl)- paraphénylènediamine, la 2-hydroxyméthyl-paraphénylènediamine, la N,N-diméthyl 3-méthylparaphénylènediamine, la N,N-(éthyl,β-hydroxyéthyl)-paraphénylènediamine, la N-(β,γ-dihydroxypropyl)-paraphénylènediamine, la N-(4'-aminophényl)-paraphénylènediamine, la N-phényl-paraphénylènediamine, la 2-β-hydroxyéthyloxy-paraphénylènediamine, la 2-β-acétylaminoéthyloxy paraphénylènediamine, la N-(β-méthoxyéthyl)-paraphénylènediamine, 2-méthyl-1-N-β-hydroxyéthyl-paraphénylènediamine, et leurs sels d'addition avec un acide.

Parmi les paraphénylènediamines de formule (I) ci-dessus, on préfère tout particulièrement la paraphénylènediamine, la paratoluylènediamine, la 2-isopropylparaphénylènediamine, la 2-β-hydroxyéthyl-paraphénylènediamine, la 2-β-hydroxyéthyloxy-paraphénylènediamine, la 2,6-diméthyl-paraphénylènediamine, la 2,6-diéthyl-paraphénylènediamine, la 2,3-diméthylparaphénylènediamine, la N,N-bis-(β-hydroxyéthyl)-paraphénylènediamine, la 2-chloro-paraphénylènediamine, et leurs sels d'addition avec un acide.
- **(II)** Selon l'invention, on entend par bases doubles, les composés comportant au moins deux noyaux aromatiques sur lesquels sont portés des groupements amino et/ou hydroxyle.
   Parmi les bases doubles utilisables à titre de bases d'oxydation dans les compositions tinctoriales conformes à l'invention, on peut notamment citer les composés répondant à la formule (II) suivante, et leurs sels d'addition avec un acide : 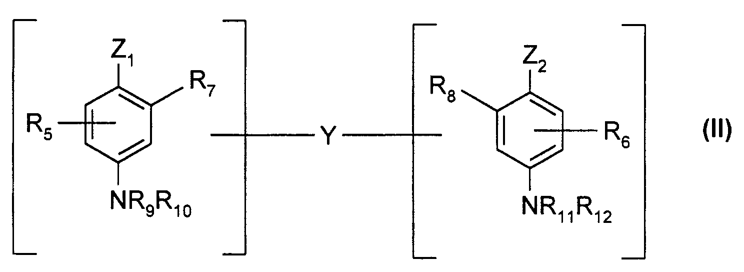 dans laquelle :
   - Z₁ et Z₂, identiques ou différents, représentent un radical hydroxyle ou -NH₂ pouvant être substitué par un radical alkyle en C₁-C₄ ou par un bras de liaison Y ;
   - le bras de liaison Y représente une chaîne alkylène comportant de 1 à 14 atomes de carbone, linéaire ou ramifiée pouvant être interrompue ou terminée par un ou plusieurs groupements azotés et/ou par un ou plusieurs hétéroatomes tels que des atomes d'oxygène, de soufre ou d'azote, et éventuellement substituée par un ou plusieurs radicaux hydroxyle ou alcoxy en C₁-C₆;
   - R₅ et R₆ représentent un atome d'hydrogène ou d'halogène, un radical alkyle en C₁-C₄, monohydroxyalkyle en C₁-C₄, polyhydroxyalkyle en C₂-C₄, aminoalkyle en C₁-C₄ ou un bras de liaison Y ;
   - R₇, R₈, R₉, R₁₀, R₁₁ et R₁₂, identiques ou différents, représentent un atome d'hydrogène, un bras de liaison Y ou un radical alkyle en C₁-C₄ ; étant entendu que les composés de formule (II) ne comportent qu'un seul bras de liaison Y par molécule.

Parmi les groupements azotés de la formule (II) ci-dessus, on peut citer notamment les radicaux amino, monoalkyl(C₁-C₄)amino, dialkyl(C₁-C₄)amino, trialkyl(C₁-C₄)amino, monohydroxyalkyl(C₁-C₄)amino, imidazolinium et ammonium.

Parmi les bases doubles de formules (II) ci-dessus, on peut plus particulièrement citer le N,N'-bis-(β-hydroxyéthyl) N,N'-bis-(4'-aminophényl) 1,3-diamino propanol, la N,N'-bis-(β-hydroxyéthyl) N,N'-bis-(4'-aminophényl) éthylènediamine, la N,N'-bis-(4-aminophényl) tétraméthylènediamine, la N,N'-bis-(β-hydroxyéthyl) N,N'-bis-(4-aminophényl) tétraméthylènediamine, la N,N'-bis-(4-méthylaminophényl) tétraméthylènediamine, la N,N'-bis-(éthyl) N,N'-bis-(4'-amino, 3'-méthylphényl) éthylènediamine, le 1,8-bis-(2,5-diaminophénoxy)-3,5-dioxaoctane, et leurs sels d'addition avec un acide.

Parmi ces bases doubles de formule (II), le N,N'-bis-(β-hydroxyéthyl) N,N'-bis-(4'-aminophényl) 1,3-diamino propanol, le 1,8-bis-(2,5-diaminophénoxy)-3,5-dioxaoctane ou l'un de leurs sels d'addition avec un acide sont particulièrement préférés.
- **(III)** les para-aminophénols répondant à la formule (III) suivante, et leurs sels d'addition avec un acide : 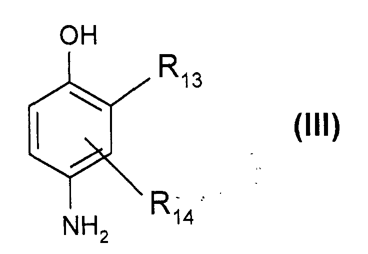 dans laquelle :
   **R**_{**13**} représente un atome d'hydrogène,un atome d'halogène tel que le fluor, un radical alkyle en C₁-C₄, monohydroxyalkyle en C₁-C₄, alcoxy(C₁-C₄)alkyle(C₁-C₄) ou aminoalkyle en C₁-C₄, ou hydroxyalkyl(C₁-C₄)aminoalkyle en C₁-C₄.
   **R**_{**14**} représente un atome d'hydrogène ou un atome d'halogène tel que le fluor, un radical alkyle en C₁-C₄, monohydroxyalkyle en C₁-C₄, polyhydroxyalkyle en C₂-C₄, aminoalkyle en C₁-C₄, cyanoalkyle en C₁-C₄ ou alcoxy(C₁-C₄)alkyle(C₁-C₄).

Parmi les para-aminophénols de formule (III) ci-dessus, on peut plus particulièrement citer le para-aminophénol, le 4-amino-3-méthyl-phénol, le 4-amino-3-fluoro-phénol, le 4-amino-3-hydroxyméthyl-phénol, le 4-amino-2-méthyl-phénol, le 4-amino-2-hydroxyméthyl-phénol, le 4-amino-2-méthoxyméthyl-phénol, le 4-amino-2-aminométhyl-phénol, le 4-amino-2-(β-hydroxyéthyl-aminométhyl)-phénol, et leurs sels d'addition avec un acide.
- **(IV)** les ortho-aminophénols utilisables à titre de bases d'oxydation dans le cadre de la présente l'invention, sont notamment choisis parmi le 2-amino-phénol, le 2-amino-1-hydroxy-5-méthyl-benzène, le 2-amino-1-hydroxy-6-méthyl-benzène, le 5-acétamido-2-amino-phénol, et leurs sels d'addition avec un acide.
- **(V)** parmi les bases hétérocycliques utilisables à titre de bases d'oxydation dans les compositions tinctoriales conformes à l'invention, on peut plus particulièrement citer les dérivés pyridiniques, les dérivés pyrimidiniques, les dérivés pyrazoliques, et leurs sels d'addition avec un acide.

Parmi les dérivés pyridiniques, on peut plus particulièrement citer les composés décrits par exemple dans les brevets GB 1 026 978 et GB 1 153 196, comme la 2,5-diamino-pyridine, la 2-(4-méthoxyphényl)amino-3-amino-pyridine, la 2,3-diamino-6-méthoxy-pyridine, la 2-(β-méthoxyéthyl)amino-3-amino-6-méthoxy pyridine, la 3,4-diamino-pyridine, et leurs sels d'addition avec un acide.

Parmi les dérivés pyrimidiniques, on peut plus particulièrement citer les composés décrits par exemple dans les brevets allemand DE 2 359 399 ou japonais JP 88-169 571 et JP 91-10659 ou demandes de brevet WO 96/15765, comme la 2,4,5,6-tétra-aminopyrimidine, la 4-hydroxy-2,5,6-triaminopyrimidine, la 2-hydroxy-4,5,6-triaminopyrimidine, la 2,4-dihydroxy-5,6-diaminopyrimidine, la 2,5,6-triaminopyrimidine, et les dérivés pyrazolo-pyrimidiniques tels ceux mentionnés dans la demande de brevet FR-A-2 750 048 et parmi lesquels on peut citer la pyrazolo-[1,5-a]-pyrimidine-3,7-diamine ; la 2,5-diméthyl-pyrazolo-[1,5-a]-pyrimidine-3,7-diamine ; la pyrazolo-[1,5-a]-pyrimidine-3,5-diamine ; la 2,7-diméthyl-pyrazolo-[1,5-a]-pyrimidine-3,5-diamine; le 3-amino-pyrazolo-[1,5-a]-pyrimidin-7-ol ; le 3-amino-pyrazolo-[1,5-a]-pyrimidin-5-ol ; le 2-(3-amino pyrazolo-[1,5-a]-pyrimidin-7-ylamino)-éthanol, le 2-(7-amino-pyrazolo-[1,5-a]-pyrimidin-3-ylamino)-éthanol, le 2-[(3-amino-pyrazolo[1,5-a]pyrimidin-7-yl)-(2-hydroxy-éthyl)-amino]-éthanol, le 2-[(7-amino-pyrazolo[1,5-a]pyrimidin-3-yl)-(2-hydroxy-éthyl)-amino]-éthanol, la 5,6-diméthyl-pyrazolo-[1,5-a]-pyrimidine-3,7-diamine, la 2,6-diméthyl-pyrazolo-[1,5-a]-pyrimidine-3,7-diamine, la 2, 5, N7, N7-tetraméthyl-pyrazolo-[1,5-a]-pyrimidine-3,7-diamine, la 3-amino-5-méthyl-7-imidazolylpropylamino pyrazolo-[1,5-a]-pyrimidine, et leurs sels d'addition et leurs formes tautomères, lorsqu'il existe un équilibre tautomérique et leurs sels d'addition avec un acide.

Parmi les dérivés pyrazoliques, on peut plus particulièrement citer les composés décrits dans les brevets DE 3 843 892, DE 4 133 957 et demandes de brevet WO 94/08969, WO 94/08970, FR-A-2 733 749 et DE 195 43 988 comme le 4,5-diamino-1-méthyl-pyrazole, le 3,4-diamino-pyrazole, le 4,5-diamino-1-(4'-chlorobenzyl)-pyrazole, le 4,5-diamino 1,3-diméthyl-pyrazole, le 4,5-diamino-3-méthyl-1-phényl pyrazole, le 4,5-diamino 1-méthyl-3-phényl-pyrazole, le 4-amino-1,3-diméthyl-5-hydrazino-pyrazole, le 1-benzyl-4,5-diamino-3-méthyl-pyrazole, le 4,5-diamino-3-tert-butyl-1-méthyl pyrazole, le 4,5-diamino-1-tert-butyl-3-méthyl-pyrazole, le 4,5-diamino-1-(β-hydroxyéthyl)-3-méthyl pyrazole, le 4,5-diamino-1-éthyl-3-méthyl-pyrazole, le 4,5-diamino-1-éthyl-3-(4'-méthoxyphényl)-pyrazole, le 4,5-diamino-1-éthyl-3-hydroxyméthyl-pyrazole, le 4,5-diamino-3-hydroxyméthyl-1-méthyl-pyrazole, le 4,5-diamino-3-hydroxyméthyl-1-isopropyl-pyrazole, le 4,5-diamino-3-méthyl-1-isopropylpyrazole, le 4-amino-5-(2'-aminoéthyl)amino-1,3-diméthyl-pyrazole, le 3,4,5-triamino-pyrazole, le 1-méthyl-3,4,5-triamino-pyrazole, le 3,5-diamino 1-méthyl-4-méthylamino-pyrazole, le 3,5-diamino-4-(β-hydroxyéthyl)amino 1-méthyl-pyrazole, et leurs sels d'addition avec un acide.

Selon la présente invention, les bases d'oxydation représentent de préférence de 0,0005 à 12% en poids environ du poids total de la composition et encore plus préférentiellement de 0,005 à 8% en poids environ de ce poids.

Les coupleurs utilisables sont ceux classiquement utilisés dans les compositions de teinture d'oxydation, c'est-à-dire des métaphénylènediamines, des métaaminophénols et des métadiphénols, les dérivés mono- ou poly-hydroxylés du naphtalène, le sésamol et ses dérivés et des composés hétérocycliques tels que par exemple les coupleurs indoliques, les coupleurs indoliniques, les coupleurs pyridiniques et leurs sels d'addition avec un acide.

Ces coupleurs peuvent notamment être choisis parmi le 2-méthyl-5-amino-phénol, le 5-N-(β-hydroxyéthyl)-amino-2-méthyl-phénol, le 3-amino-phénol, le 1,3-dihydroxybenzène, le 1,3-dihydroxy-2-méthyl-benzène, le 4-chloro-1,3-dihydroxy-benzène, le 2,4-diamino-1-(β-hydroxyéthyloxy)-benzène, le 2-amino-4-(β-hydroxyéthylamino)-1-méthoxy-benzène, le 1,3-diamino-benzène, le 1,3-bis-(2,4-diaminophénoxy)-propane, 1-amino-2-méthoxy-4,5-méthylènedioxy benzène, le sésamol, l'α-naphtol, le 6-hydroxy-indole, le 4-hydroxy-indole, le 4-hydroxy-N-méthyl-indole, la 6-hydroxyindoline, le chlorure de 3-(4-hydroxy-1-méthyl-1H-indol-5-yle-méthyl)-1-méthylpyridinium, la 2,6-dihydroxy-4-méthyl-pyridine, le 1-H-3-méthyl-pyrazole-5-one, le 1-phényl-3-méthyl-pyrazole-5-one, et leurs sels d'addition avec un acide.

Lorsqu'ils sont présents ces coupleurs représentent de préférence de 0,0001 à 10 % en poids environ du poids total de la composition tinctoriale prête à l'emploi et encore plus préférentiellement de 0,005 à 5 % en poids environ de ce poids.

D'une manière générale, les sels d'addition avec un acide utilisables dans le cadre des compositions tinctoriales de l'invention (bases d'oxydation et coupleurs) sont notamment choisis parmi les chlorhydrates, les bromhydrates, les sulfates et les tartrates, les lactates et les acétates.

La composition tinctoriale de l'invention peut encore contenir, en plus des bases d'oxydation définis ci-dessus et des éventuels coupleurs associés, des colorants directs pour enrichir les nuances en reflets. Ces colorants directs peuvent notamment alors être choisis parmi les colorants nitrés, azoïques ou anthraquinoniques, neutres, cationiques ou anioniques dans la proportion pondérale d'environ 0,001 à 20% et de préférence de 0,01 à 10% du poids total de la composition.

L'invention a également pour objet un procédé de teinture des fibres kératiniques et en particulier des fibres kératiniques humaines telles que les cheveux mettant en oeuvre la composition tinctoriale prête à l'emploi telle que définie précédemment.
Selon ce procédé, on applique sur les fibres au moins une composition tinctoriale prête à l'emploi telle que définie précédemment, pendant un temps suffisant pour développer la coloration désirée, après quoi on rince, on lave éventuellement au shampooing, on rince à nouveau et on sèche.
Le temps nécessaire au développement de la coloration sur les fibres kératiniques est généralement compris entre 3 et 60 minutes et encore plus précisément 5 et 40 minutes.

Selon une forme de réalisation particulière de l'invention, le procédé comporte une étape préliminaire consistant à stocker sous forme séparée, d'une part, une composition (A) comprenant, dans un milieu approprié pour la teinture, au moins un colorant d'oxydation tel que défini précédemment et, d'autre part, une composition (B) renfermant, dans un milieu approprié pour la teinture, au moins un système oxydant enzymatique comprenant au moins une enzyme de type oxydo-réductase à 2 électrons ou à 4 électrons ou peroxydase, et au moins un copolymère réticulé anhydride maléique / alkyl(C₁-C₅) vinyl éther, puis à procéder à leur mélange au moment de l'emploi avant d'appliquer ce mélange sur les fibres kératiniques.

Selon une autre forme de réalisation particulière de l'invention, le copolymère réticulé anhydride maléique / alkyl(C₁-C₅) vinyl éther, est incorporé dans la composition (A).

Un autre objet de l'invention est un dispositif à plusieurs compartiments ou "kit" de teinture ou tout autre système de conditionnement à plusieurs compartiments dont un premier compartiment renferme la composition (A) telle que définie ci-dessus et un second compartiment renferme la composition (B) telle que définie ci-dessus. Ces dispositifs peuvent être équipés d'un moyen permettant de délivrer sur les cheveux le mélange souhaité, tel que les dispositifs décrits dans le brevet FR-2 586 913 au nom de la demanderesse.

La présente invention a également pour objet un nouveau procédé de traitement des fibres kératiniques, en particulier des cheveux humains, en vue d'obtenir une déformation permanente de ces derniéres, en particulier sous la forme de cheveux permanentés, ce procédé comprenant les étapes suivantes : (i) on applique sur la fibre kératinique à traiter une composition réductrice, la fibre kératinique étant mise sous tension mécanique avant, pendant, ou après ladite application, (ii) on rince éventuellement la fibre kératinique, (iii) on applique sur la fibre kératinique éventuellement rincée une composition oxydante telle que définie ci-dessus, (iv) on rince éventuellement à nouveau la fibre kératinique.

La première étape (i) de ce procédé consiste à appliquer sur les cheveux une composition réductrice. Cette application se fait mèche par mèche ou globalement.

La composition réductrice comprend par exemple au moins un agent réducteur, qui peut être en particulier choisi parmi l'acide thioglycolique, la cystéine, la cystéamine, le thioglycolate de glycérol, l'acide thiolactique, ou les sels des acides thiolactique ou thioglycolique.

L'habituelle étape de mise sous tension des cheveux sous une forme correspondant à la forme finale désirée pour ces derniers (boucles par exemple) peut être mise en oeuvre par tout moyen, mécanique notamment, approprié et connu en soi pour maintenir sous tension des cheveux, tels que par exemple rouleaux, bigoudis et analogues.

Les cheveux peuvent également être mis en forme sans l'aide de moyens extérieurs, simplement avec les doigts.
Avant de procéder à l'étape (ii) suivante facultative de rinçage, il convient, de manière classique, de laisser reposer pendant quelques minutes, généralement entre 5 minutes et une heure, de préférence entre 10 et 30 minutes, la chevelure sur laquelle a été appliquée la composition réductrice, et ceci de façon à bien laisser le temps au réducteur d'agir correctement sur les cheveux.
Dans la deuxième étape, facultative, du procédé (étape (ii)), les cheveux imprégnés de la composition réductrice sont donc ensuite rincés soigneusement par une composition aqueuse.
Puis, dans une troisième étape (étape (iii)), on applique sur les cheveux ainsi rincés la composition oxydante de l'invention, dans le but de fixer la nouvelle forme imposée aux cheveux.
Comme dans le cas de l'application de la composition réductrice, la chevelure sur laquelle a été appliquée la composition oxydante est ensuite, de manière classique, laissée dans une phase de repos ou d'attente qui dure quelques minutes, généralement entre 3 et 30 minutes, de préférence entre 5 et 15 minutes.
Si la tension des cheveux était maintenue par des moyens extérieurs, on peut retirer de la chevelure ces derniers (rouleaux, bigoudis et analogues) avant ou après l'étape de fixation.

Enfin, dans la dernière étape du procédé selon l'invention (étape (iv)), étape facultative également, les cheveux imprégnés de la composition oxydante sont rincés soigneusement, généralement à l'eau.
On obtient finalement une chevelure facile à démêler, douce. Les cheveux sont ondulés.

La composition oxydante selon l'invention peut également être utilisée dans un procédé de décoloration des fibres kératiniques, et en particulier des cheveux humains.

Le procédé de décoloration selon l'invention comprend une étape d'application sur les fibres kératiniques d'une composition oxydante selon l'invention en présence ou non d'un oxydant auxiliaire. Classiquement, une deuxième étape du procédé de décoloration selon l'invention est une étape de rinçage des fibres kératiniques.

Le milieu approprié pour les fibres kératiniques (ou support) des compositions tinctoriales prêtes à l'emploi et des compositions oxydantes utilisées pour la déformation permanente ou la décoloration des fibres kératiniques conformes à l'invention est généralement constitué par de l'eau ou par un mélange d'eau et d'au moins un solvant organique pour solubiliser les composés qui ne seraient pas suffisamment solubles dans l'eau. A titre de solvant organique, on peut par exemple citer les alcanols en C₁-C₄, tels que l'éthanol et l'isopropanol ; les polyols et leurs éthers comme le glycérol, le 2-butoxyéthanol, le propylèneglycol, le monométhyléther de propylèneglycol, le monoéthyléther et le monométhyléther du diéthylèneglycol, ainsi que les alcools aromatiques comme l'alcool benzylique ou le phénoxyéthanol, les produits analogues et leurs mélanges.

Les solvants peuvent être présents dans des proportions de préférence comprises entre 1 et 40% en poids environ par rapport au poids total de la composition tinctoriale, et encore plus préférentiellement entre 5 et 30% en poids environ.

Le pH des compositions tinctoriales prêtes à l'emploi et des compositions oxydantes utilisées pour la déformation permanente ou la décoloration des fibres kératiniques conformes à l'invention est choisi de telle manière que l'activité enzymatique de l'oxydo-réductase à 2 électrons ou à 4 électrons ou de la peroxydase ne soit pas altérée. Il est généralement compris entre 5 et 11 environ, et de préférence entre 6,5 et 10 environ. Il peut être ajusté à la valeur désirée au moyen d'agents acidifiants ou alcalinisants habituellement utilisés en teinture des fibres kératiniques.

Parmi les agents acidifiants, on peut citer, à titre d'exemple, les acides minéraux ou organiques comme l'acide chlorhydrique, l'acide orthophosphorique, l'acide sulfurique, les acides carboxyliques comme l'acide acétique, l'acide tartrique, l'acide citrique, l'acide lactique, les acides sulfoniques.

Parmi les agents alcalinisants on peut citer, à titre d'exemple, l'ammoniaque, les carbonates alcalins, les alcanolamines telles que les mono-, di- et triéthanolamines, le 2-méthyl 2-amino propanol ainsi que leurs dérivés, les hydroxydes de sodium ou de potassium et les composés de formule (IV) suivante : 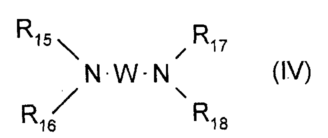 dans laquelle W est un reste propylène éventuellement substitué par un groupement hydroxyle ou un radical alkyle en C₁-C₄ ; R₁₅, R₁₆, R₁₇ et R₁₈, identiques ou différents, représentent un atome d'hydrogène, un radical alkyle en C₁-C₄ ou hydroxyalkyle en C₁-C₄.

Les compositions tinctoriales prêtes à l'emploi et les compositions oxydantes pour la déformation permanente ou la décoloration des fibres kératiniques conformes à l'invention peuvent également comprendre divers adjuvants utilisés classiquement dans les compositions pour la teinture, la déformation permanente ou la décoloration des cheveux, tels que des agents tensio-actifs anioniques, non-ioniques, amphotères, zwittérioniques ou leurs mélanges, des polymères anioniques autres que ceux de l'invention, des polymères non-ioniques, des polymères amphotères, des polymères zwittérioniques ou des mélanges de polymères, des agents épaississants minéraux ou organiques, des agents antioxydants, des agents de pénétration, des agents séquestrants, des parfums, des tampons, des agents dispersants, des agents de conditionnement tels que par exemple des silicones, des agents filmogènes, des agents conservateurs, des agents opacifiants.

Bien entendu, l'homme de l'art veillera à choisir ce ou ces éventuels composés complémentaires de manière telle que les propriétés avantageuses attachées intrinsèquement aux compositions conformes à l'invention ne soient pas, ou substantiellement pas, altérées par la ou les adjonctions envisagées.

Les compositions tinctoriales prêtes à l'emploi et les compositions oxydantes utilisées pour la déformation permanente ou la décoloration des fibres kératiniques conformes à l'invention, peuvent se présenter sous des formes diverses, telles que sous forme de liquides, de crèmes, de gels, éventuellement pressurisés, ou sous toute autre forme appropriée pour réaliser une teinture, une déformation permanente ou une décoloration des fibres kératiniques, et notamment des cheveux humains.

Dans le cas d'une composition tinctoriale prête à l'emploi, le ou les colorants d'oxydation et la ou les oxydo-réductases à 2 électrons ou à 4 électrons ou peroxydases sont présents au sein de ladite composition qui doit être exempte d'oxygène gazeux, de manière à éviter toute oxydation prématurée du ou des colorants d'oxydation au cours du stockage de ladite composition.

Les exemples suivants illustrent l'invention sans présenter un caractère limitatif.

### EXEMPLE 1 :

On a étudié la conservation de l'activité enzymatique de l'uricase, d'une part, dans un milieu contenant un épaississant de l'art antérieur, l'ACULYN 22, et d'autre part, dans le milieu selon la présente invention, c'est-à-dire avec du STABILEZE QM.
On a ainsi comparé une composition selon l'invention B à une composition selon l'art antérieur A (quantités exprimées en grammes de Matière Active).
Les quantités d'ACULYN 22 et de STABILEZE QM ont été ajustées pour obtenir un épaississement identique et cosmétiquement acceptable des compositions au final.

| | **B** | **A** |
|---|---|---|
| | Composition selon l'invention | Composition selon l'art antérieur |
| STABILEZE QM (I.S.F) | 1 | |
| ACULYN 22 (ROHM & HAAS) | | 2,5 |
| Mono-oléate de polyglycérol (10 moles) | 1 | 1 |
| N-acétyl-L-cystéine | 0,1 | 0,1 |
| 2-amino-2-méthyl-1-propanol q.s pH | 9,5 | 9,5 |
| Uricase | 20000 U | 20000 U |
| Acide urique | 1 | 1 |
| Eau déminéralisée q.s.p | 100 | 100 |

L'activité uricase dans le support oxydant est suivie par oxymétrie au moyen d'une électrode de Clark qui enregistre la diminution de la concentration en oxygène dissous lors de l'oxydation de l'acide urique en allantoïne par l'uricase :

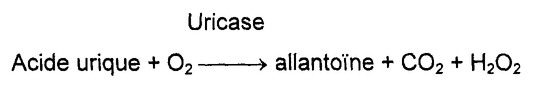

La vitesse de consommation de l'oxygène dissous dans le support est comparée à celle obtenue dans des solutions étalon d'uricase ce qui permet de déduire l'activité enzymatique dans le milieu étudié.

### Résultats

Pour chaque mesure d'activité, 3 dosages ont été réalisés.

Les résultats ont été donnés en terme de moyenne±intervalle de confiance à 5% dans le tableau (I) donné ci-après.

Il en résulte que le STABILEZE QM est significativement supérieur à l'état de la technique, en terme de conservation de l'activité uricase.

### EXEMPLE 2, 3, 4 :

On a préparé les compositions de teinture prêtes à l'emploi suivantes :

### (quantités exprimées en grammes)

| **Compositions** | **2** | **3** | **4** |
|---|---|---|---|
| STABILEZE QM (I.S.F) | 1 | 1 | 1 |
| Paraphénylènediamine | 0,324 | 0,324 | 0,324 |
| 2-méthyl-5-amino-phénol | 0,369 | | |
| 2,4-diamino-1-(β-hydroxyéthyloxy)-benzène 2HCl | | 0,723 | |
| 1-amino-2-méthoxy-4,5-méthylènedioxy benzène | | | 0,6108 |
| Mono-oléate de polyglycérol (10 moles) | 1 | 1 | 1 |
| N-acétyl-L-cystéine | 0,1 | 0,1 | 0,1 |
| 2-amino-2-méthyl-1-propanol q.s pH | 9,5 | 9,5 | 9,5 |
| Uricase | 1 soit 20000 U | 1 soit 20000 U | 1 soit 20000 U |
| Acide urique | 1 | 1 | 1 |
| Eau déminéralisée q.s.p | 100 | 100 | 100 |

Parallèlement, on a réalisé des compositions de l'art antérieur 5, 6 et 7, ne différant des compositions 2, 3 et 4 ci-dessus que par la présence de 2,5% d'ACULYN 22 au lieu de 1% de STABILEZE.

Chaque composition tinctoriale prête à l'emploi 2, 3, 4, 5, 6 et 7 a été appliquée sur des mèches de cheveux gris naturels à 90 % de blancs, et sur des mèches de cheveux gris permanentés à 90 % de blancs pendant 30 minutes à température ambiante. Les cheveux ont ensuite été rincés, lavés avec un shampooing standard, puis séchés.

Résultats : les mèches de cheveux obtenues à partir des compositions 2, 3, 4 ont été teintes de façon homogène dans des nuances peu sélectives et aussi puissantes et aussi chromatiques que les mèches de cheveux des compositions de l'art antérieur 5, 6 et 7.

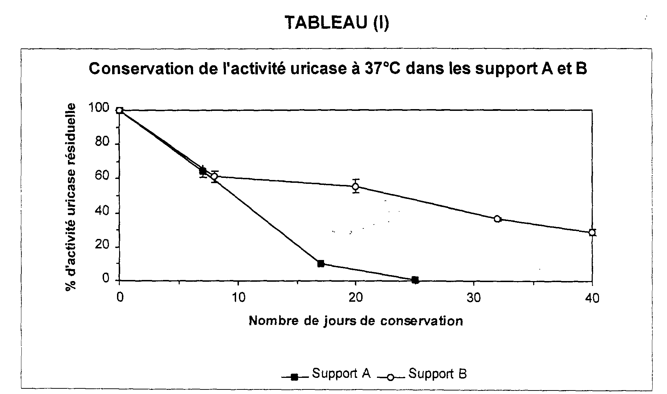

## Revendications

1. Composition oxydante cosmétique destinée au traitement des fibres kératiniques, en particulier des fibres kératiniques humaines et plus particulièrement les cheveux humains, comprenant dans un milieu pour les fibres kératiniques :
(a) au moins un système oxydant enzymatique comprenant au moins une enzyme de type oxydo-réductase à 2 électrons ou à 4 électrons ou peroxydase,
(b) au moins un copolymère réticulé anhydride maléique / alkyl(C₁-C₅) vinyl éther.

2. Composition selon la revendication 1, **caractérisée par le fait que** les oxydo-réductases à 2 électrons sont utilisées avec un donneur pour la ou lesdites enzymes et sont choisies parmi les pyranose oxydases, les glucose oxydases, les glycérol oxydases, les lactate oxydases, les pyruvate oxydases, les uricases, les choline oxydases, les sarcosine oxydases, les bilirubine oxydases et les aminoacides oxydases.

3. Composition selon la revendication 2, **caractérisée par le fait que** l'oxydo-réductase à 2 électrons est choisie parmi les uricases d'origine animale, microbiologique ou biotechnologique.

4. Composition selon les revendications 2 ou 3, **caractérisée par le fait que** le donneur pour ladite oxydo-réductase à 2 électrons est choisi parmi l'acide urique et ses sels.

5. Composition selon la revendication 1, **caractérisée par le fait que** les oxydo-réductases à 4 électrons sont choisies parmi les laccases, les tyrosinases, les catéchol oxydases et les polyphénols oxydases.

6. Composition selon la revendication 5, **caractérisée par le fait que** les oxydo-réductases à 4 électrons sont choisies parmi les laccases d'origine végétale, d'origine animale, d'origine fongique ou d'origine bactérienne et parmi les laccases obtenues par biotechnologie.

7. Composition selon la revendication 6, **caractérisée par le fait que caractérisée par le fait que** la laccase est d'origine végétale et choisie parmi les laccases présentes dans les extraits d'Anacardiacées ; de Podocarpacées ; de Rosmarinus off. ; de Solanum tuberosum ; d'Iris sp. ; de Coffea sp. ; de Daucus carrota ; de Vinca minor ; de Persea americana ; de Catharenthus roseus ; de Musa sp. ; de Malus pumila ; de Gingko biloba ; de Monotropa hypopithys (sucepin); d'Aesculus sp.; d'Acer pseudoplatanus ; de Prunus persica et de Pistacia palaestina.

8. Composition selon la revendication 6, **caractérisée par le fait que** la laccase est d'origine fongique ou obtenue par biotechnologie.

9. Composition selon la revendication 8, **caractérisée par le fait que** la laccase est choisie parmi les laccases issues de Polyporus versicolor, de Rhizoctonia praticola, de Rhus vernicifera, de Scytalidium, de Polyporus pinsitus, de Myceliophtora thermophila, de Rhizoctonia solani, de Pyricularia orizae, de Trametes versicolor, de Fomes fomentarius, de Chaetomium thermophile, de Neurospora crassa, de Colorius versicol, de Botrytis cinerea, de Rigidoporus lignosus, de Phellinus noxius, de Pleurotus ostreatus, d'Aspergillus nidulans, de Podospora anserina, d'Agaricus bisporus, de Ganoderma lucidum, de Glomerella cingulata, de Lactarius piperatus, de Russula delica, d'Heterobasidion annosum, de Thelephora terrestris, de Cladosporium cladosporioides, de Cerrena unicolor, de Coriolus hirsutus, de Ceriporiopsis subvermispora, de Coprinus cinereus, de Panaeolus papilionaceus, de Panaeolus sphinctrinus, de Schizophyllum commune, de Dichomitus squalens, et de leurs variantes.

10. Composition selon la revendication 1, **caractérisée par le fait que** les peroxydases sont des peroxydases simplex.

11. Composition selon la revendication 1, **caractérisée par le fait que** la ou les peroxydases sont des catalases.

12. Composition selon la revendication 1, **caractérisée par le fait que** la ou les peroxydases sont utilisées avec un donneur pour la ou lesdites enzymes et sont choisies parmi les NADH peroxydases, les acides gras peroxydases, les NADPH peroxydases, les cytochrome-c peroxydases, les iodures peroxydases, les chlorures peroxydases, les L. ascorbates, les glutathion peroxydases .

13. Composition selon l'une quelconque des revendications 1, 10 à 12, **caractérisée par le fait que** la ou les peroxydases sont d'origine animale, végétale, fongique, bactérienne ou obtenues par biotechnologie.

14. Composition selon la revendication 13, **caractérisée par le fait que** la ou les peroxydases sont issues de la pomme, de l'abricot, de l'orge, du radis noir, de la betterave, du chou, de la carotte, du maïs, du coton, de l'ail, du raisin, de la menthe, de la rhubarbe, du soja, de l'épinard, du coprin, du lait de bovin, des microorganismes du type Acetobacter peroxidans, Staphylococcus faecalis, Arthromyces ramosus.

15. Composition selon l'une quelconque des revendications 1 à 9, **caractérisée par le fait que** les oxydo-réductases à 2 électrons ou à 4 électrons représentent de 0,01 à 20% en poids du poids total de la composition.

16. Composition selon la revendication 15, **caractérisée par le fait que** les oxydo-réductases à 2 électrons ou à 4 électrons représentent de 0,1 à 5% en poids du poids total de la composition.

17. Composition selon l'une quelconque des revendications 1, 10 à 14, **caractérisée par le fait que** la ou les peroxydases représentent de 0,0001 à 20% en poids du poids total de la composition.

18. Composition selon la revendication 17, **caractérisée par le fait que** la ou les peroxydases représentent de 0,001 à 10% en poids du poids total de la composition.

19. Composition selon l'une quelconque des revendications 1 à 18, **caractérisée par le fait que** le copolymère réticulé anhydride maléique / alkyl(C₁-C₅) vinyl éther, en solution dans l'eau à une concentration de 0,5 à 1%, hydrolysé et neutralisé, à un pH compris entre 5 et 10, présente une viscosité mesurée à 25°C au viscosimètre BROOKFIELD RTV, broche 7 à 20 tours/minute, comprise entre 45000 et 70000 mPa.s.

20. Composition selon la revendication 19, **caractérisée par le fait que** la taille des particules du copolymère réticulé anhydride maléique / alkyl(C₁-C₅) vinyl éther est inférieure à 850 microns.

21. Composition selon la revendication 20, **caractérisée par le fait que** la taille des particules du copolymère réticulé anhydride maléique / alkyl(C₁-C₅) vinyl éther est inférieure à 75 microns.

22. Composition selon la revendication 21, **caractérisée par le fait que** la taille des particules du copolymère réticulé anhydride maléique / alkyl(C₁-C₅) vinyl éther est comprise entre 1 et 15 microns.

23. Composition selon l'une quelconque des revendications précédentes, **caractérisée par le fait que** le copolymère réticulé anhydride maléique / alkyl(C₁-C₅) vinyl éther est un copolymère anhydride maléique / méthyl vinyl éther réticulé par le 1,9-décadiène.

24. Composition selon l'une quelconque des revendications précédentes, **caractérisée par le fait que** la concentration en copolymère réticulé anhydride maléique / alkyl(C₁-C₅) vinyl éther est comprise entre 0,001 % et 10% en poids par rapport au poids total de la composition.

25. Composition selon la revendication 24, **caractérisée par le fait que** la concentration en copolymère réticulé anhydride maléique / alkyl(C₁-C₅) vinyl éther est comprise entre 0,01 et 5% en poids par rapport au poids total de la composition.

26. Composition selon l'une quelconque des revendications précédentes, prête à l'emploi, pour la teinture d'oxydation des fibres kératiniques et en particulier des fibres kératiniques humaines telles que les cheveux, **caractérisée par le fait qu'**elle comprend en outre au moins un colorant d'oxydation.

27. Composition selon la revendication 26, **caractérisée par le fait que** le colorant d'oxydation est une base d'oxydation choisie parmi les ortho- et paraphénylènediamines, les bases doubles, les ortho- et para-aminophénols, les bases hétérocycliques, ainsi que les sels d'addition de ces composés avec un acide.

28. Composition selon la revendication 26 ou 27, **caractérisée par le fait que** la ou les bases d'oxydation sont présentes dans des concentrations allant de 0,0005 à 12% en poids par rapport au poids total de la composition.

29. Composition selon la revendication 26, **caractérisée par le fait que** le colorant d'oxydation est un coupleur choisi parmi les métaphénylènediamines, les méta-aminophénols, les métadiphénols, les dérivés mono- ou poly-hydroxylés du naphtalène, le sésamol et ses dérivés, les coupleurs hétérocycliques, et les sels d'addition de ces composés avec un acide.

30. Composition selon la revendication 29, **caractérisée par le fait que** le ou les coupleurs sont présents dans des concentrations allant de 0,0001 à 10% en poids par rapport au poids total de la composition.

31. Composition selon l'une quelconque des revendications 26 à 30, **caractérisée par le fait que** les sels d'addition avec un acide des bases d'oxydation et des coupleurs sont choisis parmi les chlorhydrates, les bromhydrates, les sulfates, les tartrates, les lactates et les acétates.

32. Composition selon l'une quelconque des revendications 26 à 31, **caractérisée par le fait qu'**elle contient en outre des colorants directs dans la proportion pondérale de 0,001 à 20% en poids par rapport au poids total de la composition.

33. Composition selon l'une quelconque des revendications précédentes, **caractérisée par le fait que** le milieu approprié pour les fibres kératiniques (ou support) est constitué par de l'eau ou par un mélange d'eau et d'au moins un solvant organique.

34. Composition selon la revendication 33, **caractérisée par le fait que** les solvants organiques peuvent être présents dans des proportions allant 1 à 40% en poids par rapport au poids total de la composition, et de préférence de 5 à 30% en poids.

35. Composition selon l'une quelconque des revendications 1 à 34, **caractérisée par le fait que** le pH varie de 5 à 11, et de préférence de 6,5 à 10.

36. Composition selon l'une quelconque des revendications précédentes, **caractérisée par le fait qu'**elle comprend en plus au moins un adjuvant cosmétique utilisé classiquement dans les compositions pour la teinture, la déformation permanente ou la décoloration des cheveux, choisi dans le groupe constitué par des agents tensio-actifs anioniques, non-ioniques, amphotères, zwittérioniques ou leurs mélanges, des polymères anioniques autres que ceux de l'invention, des polymères non-ioniques, des polymères amphotères, des polymères zwittérioniques ou des mélanges de polymères, des agents épaississants minéraux ou organiques, des agents antioxydants, des agents de pénétration, des agents séquestrants, des parfums, des tampons, des agents dispersants, des agents de conditionnement, des agents filmogènes, des agents conservateurs, des agents opacifiants.

37. Procédé de teinture des fibres kératiniques et en particulier des fibres kératiniques humaines telles que les cheveux, **caractérisé par le fait qu'**on applique sur lesdites fibres au moins une composition tinctoriale prête à l'emploi telle que définie dans l'une quelconque des revendications 26 à 36, pendant un temps suffisant pour développer la coloration désirée.

38. Procédé selon la revendication 37, **caractérisé par le fait qu'**il comporte une étape préliminaire consistant à stocker sous forme séparée, d'une part, une composition (A) comprenant, dans un milieu approprié pour la teinture, au moins un colorant d'oxydation tel que défini dans l'une quelconque des revendications 26 à 31 et d'autre part, une composition (B) comprenant, dans un milieu approprié pour les fibres kératiniques, au moins une enzyme en la présence éventuelle d'au moins un donneur pour ladite enzyme puis à procéder à leur mélange au moment de l'emploi avant d'appliquer ce mélange sur les fibres kératiniques ; la composition (A) ou la composition (B) contenant le copolymère réticulé anhydride maléique / alkyl(C₁-C₅) vinyl éther tel que défini à l'une quelconque des revendications 19 à 25.

39. Dispositif à plusieurs compartiments ou "kit" de teinture, **caractérisé par le fait qu'**il comporte un premier compartiment renfermant la composition (A) telle que définie dans la revendication 38 et un second compartiment renfermant la composition (B) telle que définie dans la revendication 38.

40. Procédé de traitement des fibres kératiniques, en particulier des cheveux humains, en vue d'obtenir une déformation permanente de ces dernières, sous la forme de cheveux permanentés, comprenant les étapes suivantes : (i) on applique sur les fibres kératiniques à traiter une composition réductrice, la fibre kératinique étant mise sous tension mécanique avant, pendant, ou après ladite application, (ii) on rince éventuellement la fibre kératinique, (iii) on applique sur la fibre kératinique éventuellement rincée une composition oxydante telle que définie dans l'une quelconque des revendications 1 à 25 et 33 à 36, (iv) on rince éventuellement à nouveau la fibre kératinique.

41. Procédé de traitement des fibres kératiniques, en particulier des cheveux humains, en vue d'obtenir une décoloration de ces dernières comprenant l'application d'une composition oxydante telle que définie dans l'une quelconque des revendications 1 à 25, 33 à 36 contenant éventuellement un agent oxydant auxiliaire puis une étape de rinçage des fibres kératiniques.

42. Procédé pour améliorer la conservation de l'activité enzymatique d'une composition oxydante destinée à la teinture, la déformation permanente ou la décoloration des fibres kératiniques, en particulier des cheveux humains, et comprenant à titre de système oxydant au moins une oxydo-réductase à 2 ou à 4 électrons ou une peroxydase, **caractérisé par le fait qu'**on ajoute à ladite composition oxydante une quantité efficace d'un copolymère réticulé anhydride maléique / alkyl(C₁-C₅) vinyl éther.

43. Procédé selon la revendication 42 **caractérisé par le fait que** le système oxydant comprend au moins une uricase et de l'acide urique, et que l'on ajoute à ladite composition oxydante une quantité efficace d'un copolymère anhydride maléique / méthyl vinyl éther, réticulé par du 1,9-décadiène.
